# EUROPEAN PATENT APPLICATION

(11) **EP 3 686 584 A1**
(43) Date of publication of application: **29.07.2020**
(21) Application number: 20152683.7
(22) Date of filing: 20.01.2020
(51) Int. Cl.: G01N 27/30

(54) **METHOD FOR MANUFACTURING A WORKING ELECTRODE OF ELECTROCHEMICAL SENSOR AND PRODUCT THEREOF**

(30) Priority: 23.01.2019 TW 108102558
(71) Applicant: Phoenix Silicon International Corp., Hsinchu 300 (TW)
(72) Inventor: TSAI, HSIN-CHUAN, 300 Hsinchu (TW); LIAO, HSUEH-CHUAN, 300 Hsinchu (TW); HUANG, HIS-CHE, 300 Hsinchu (TW)
(74) Representative: Lermer, Christoph

(57) **Abstract**

A method for manufacturing a working electrode of electrochemical sensor comprises the steps of: step S1, providing a substrate (10); step S2, forming a wavy pattern (103) on the substrate (10); and step S3, disposing a conductive substance on the wavy pattern (103). A working electrode of electrochemical sensor is also disclosed.

## Description

### TECHNICAL FIELD

The present invention relates to a method for manufacturing a working electrode of electrochemical sensor and product thereof, and more particularly to a manufacturing method for improving the precision of existing three working electrodes of an electrochemical sensor.

### BACKGROUND

The popularity of various medical detection devices has been increasing in resent years. The medical detection devices can be rapid detection near to the patient in hospital bed or in clinic. Such rapid detection requires not only high sensitivity and high selectivity, but also small, inexpensive and easy to operate.

The most common example of the medical detection devices is the blood glucose meter. Most of the blood glucose meters on the market use three-electrode disposable test strips. The three-electrode disposable test strips are made with screen printing technology. However, due to the poor precision of three-electrode test strips using screen printing technology, accurate blood glucose levels cannot be detected. The requirements for accuracy of the medical detection devices are increasingly demanding.

### SUMMARY

An objective of the present invention is to solve the above-mentioned problems and to provide a method for manufacturing a working electrode of electrochemical sensor and product thereof, which can improve the precision of existing three working electrodes of electrochemical sensor. The surface of the working electrode has a wavy pattern to increase the detection region. The present invention can be used to immobilize enzymes, antibodies or nucleic acids on a working electrode for bio-sensing in various fields.

The present invention achieves the above-indicated objective by providing a method for manufacturing a working electrode of electrochemical sensor. The method comprises the steps of: step S1, providing a substrate; step S2, forming a wavy pattern on the substrate; and step S3, disposing a conductive substance on the wavy pattern.

In an embodiment of the present invention, a protective layer is formed on one side of the substrate.

In an embodiment of the present invention, a plurality of openings are formed on the protective layer of the substrate using lithography and dry etching, in step S1.

In an embodiment of the present invention, a plurality of grooves are formed on the substrate through the plurality of openings using isotropic etching , in step S1. The plurality of grooves are semicircular grooves. Or, a plurality of grooves are formed on the substrate through the plurality of openings using anisotropic etching. The plurality of grooves are inverted triangular grooves with acute angles. The edges and the bottoms of the grooves have at least one acute angle.

In an embodiment of the present invention, the protective layer is removed, in step S1. The substrate is then immersed in an isotropic etching solution, and after being immersed for a few seconds, the acute angles of the inverted triangular grooves and the grooves are formed into rounded corners. Alternatively, the substrate is treated with thermal oxidation to form the acute angles of the inverted triangular grooves and the grooves into rounded corners. The grooves with the rounded corners form the wavy pattern.

In an embodiment of the present invention, a conductive layer is formed on the wavy pattern of the substrate using evaporating or sputtering of physical vapor deposition, in step S3. A colloidal metal solution with conductive particles and colloidal solution is disposed on the conductive layer of the substrate, wherein the conductive particles are formed on the conductive layer after the colloidal solution dried.

The present invention further discloses a working electrode of electrochemical sensor. The working electrode of electrochemical sensor comprises: a substrate; a wavy pattern formed on the substrate; and a conductive substance disposed on the wavy pattern.

In an embodiment of the present invention, the wavy pattern includes a plurality of grooves formed on the substrate. Each of the grooves has rounded corners at the edge and bottom of the each groove.

In an embodiment of the present invention, the conductive substance have a conductive layer. The conductive layer is disposed on a side of the substrate with the grooves. Or, the conductive substance includes a conductive layer and a plurality of conductive particles. The conductive layer is disposed on a side of the substrate with the grooves. The conductive layer and the conductive particles are sequentially formed on the substrate.

In summary, the working electrode of the present invention has high precision and uses metal. The working electrode having nanometer particles of metal is fabricated into a chip. The surface of the detection region of the chip has a wavy pattern. The wavy pattern can increase the detection region to improve the accuracy of the detection. The working electrode of the present invention can be adhered to existing low-cost disposable test strips, and has a low-cost competitive advantage.

### BRIEF DESCRIPTION OF THE DRAWINGS

FIG. 1 is a flow chart of a method for manufacturing a working electrode of electrochemical sensor of the present invention.
FIG. 2 is a schematic view of the substrate having openings of the Embodiment 1.
FIG. 3 is a schematic view of the substrate having a plurality of grooves of the Embodiment 1.
FIG. 4 is a schematic view of the substrate with grooves and without a protective layer of the Embodiment 1.
FIG. 5 is a schematic view of the substrate having a wavy pattern of the Embodiment 1.
FIG. 6 is a schematic view of the substrate having a conductive layer of the Embodiment 1.
FIG. 7 is a schematic view of the substrate having the conductive layer and conductive particles of the Embodiment 3.
FIG. 8 is a schematic view of the substrate having openings of the Embodiment 2.
FIG. 9 is a schematic view of the substrate having a plurality of grooves of the Embodiment 2.
FIG. 10 is a schematic view of the substrate with grooves and without a protective layer of the Embodiment 2.
FIG. 11 is a schematic view of the substrate having a wavy pattern of the Embodiment 2.
FIG. 12 is a schematic view of the substrate having a conductive layer of the Embodiment 2.
FIG. 13 is a schematic view of the substrate having the conductive layer and conductive particles of the Embodiment 2.

### DETAILED DESCRIPTION

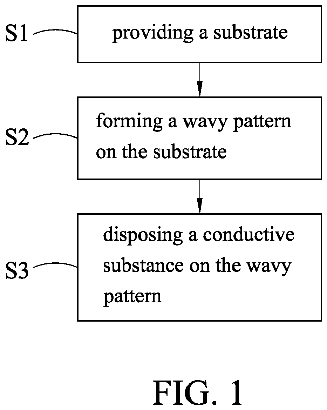

Embodiment 1: FIG. 1 is a flow chart of a method for manufacturing a working electrode of electrochemical sensor of the present invention. As shown in FIG. 1, a method for manufacturing a working electrode of electrochemical sensor of the Embodiment 1 comprises the following steps.

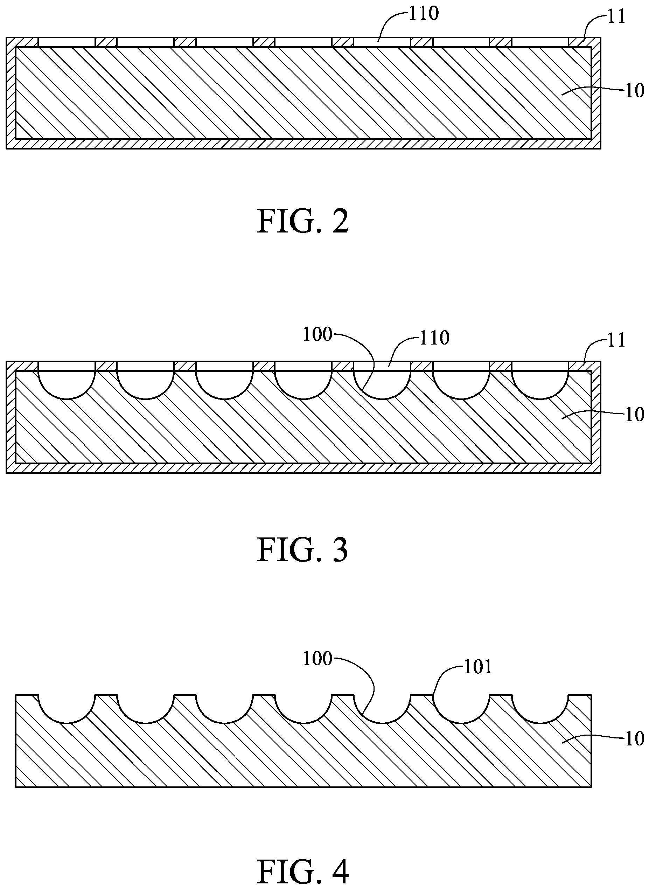

In step S1, providing a substrate, a substrate 10 is provided. A protective layer 11 is formed on one side of the substrate 10, as shown in FIG. 2. The substrate 10 is a silicon wafer having oxide or nitride with 1 to 100 ohm-cm resistivity.

In step S2, forming a wavy pattern on the substrate, a wavy pattern is formed on the substrate 10, as shown in FIG. 2. A plurality of openings 110 are formed on the protective layer 11 of the substrate 10 using lithography and dry etching.

A plurality of grooves 100 are formed on the substrate 10 through the plurality of openings 110 using isotropic etching, as shown in FIG. 3. The plurality of grooves 100 are semicircular grooves. An etching solution of the isotropic etching is 0.5-1.5 servings of 44-54 wt% hydrogen fluoride HF, 2.5-3.5 servings of 65-75 wt% nitric acid HNO₃ at a temperature between 20 and 30 °C. Preferably, 1 serving of 49% hydrogen fluoride HF and 3 servings of 70% nitric acid HNO₃, etching temperature 25 °C.

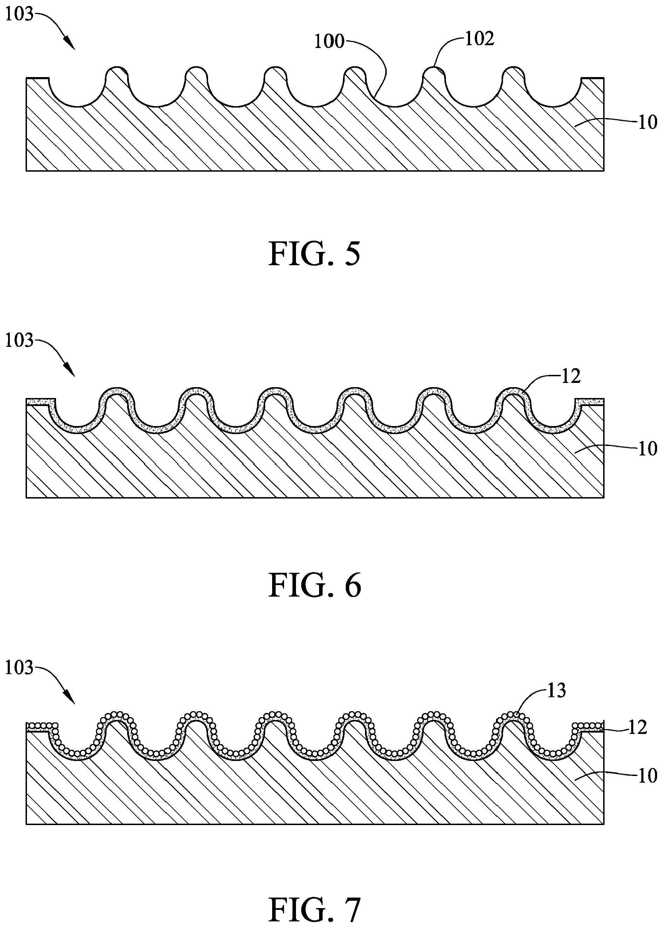

As shown in FIG. 4 and FIG. 5, the protective layer 11 is removed. Each of the grooves 100 has acute angles 101 at the edge of the each groove 100. The substrate 10 is then immersed in the isotropic etching solution, and after being immersed for a few seconds, the acute angles 101 are formed into rounded corners 102. Alternatively, the substrate 10 is treated with thermal oxidation to form the acute angles 101 into rounded corners 102. The grooves 100 with the rounded corners 102 form the wavy pattern 103.

In step S3, disposing a conductive substance on the wavy pattern, a conductive substance is disposed on the wavy pattern 103 using evaporating or sputtering of physical vapor deposition (PVD). A conductive layer 12 is formed on the wavy pattern 103 of the substrate 10 using evaporating or sputtering of physical vapor deposition, as shown in FIG. 6. The conductive layer 12 can be a chrome-copper (Cr-Cu) layer.

Embodiment 2: Referring to FIGs. 1 and 7, a method for manufacturing a working electrode of electrochemical sensor of the Embodiment 2 is based on the Embodiment 1 of the present invention described above, so the same components use the same symbols.

In the Embodiment 2, a colloidal metal solution with conductive particles 13 and colloidal solution is disposed on the conductive layer 12 of the substrate 10, wherein the conductive particles 13 are formed on the conductive layer 12 after the colloidal solution dried.

The conductive particles 13 of the colloidal metal solution can generally be prepared by reducing Gold(III) chloride trihydrate solution with sodium citrate. The conductive particles 13 can be nanometer particles of metal having a size of from 1 to 100 nm. Where the nanometer particles of metal are nanometer particles of gold, the equation is:

HAuCl₄ + Na₃C₆H₅O₇ → nano Au

As shown in FIG. 1, a method for manufacturing a working electrode of electrochemical sensor of the Embodiment 2 comprises the following steps.

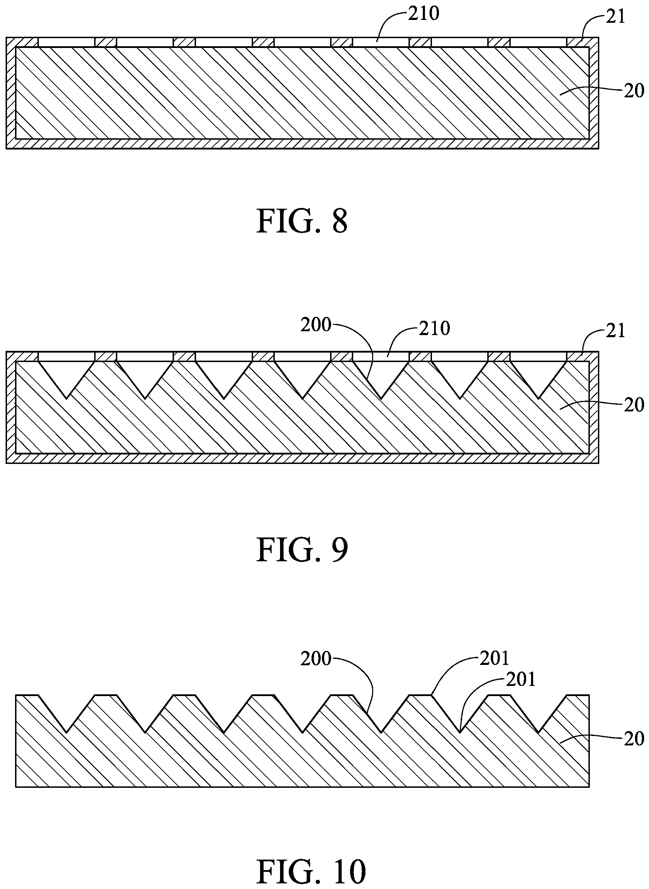

In step S1, providing a substrate, a substrate 20 is provided. A protective layer 21 is formed on one side of the substrate 20, as shown in FIG. 8. The substrate 20 is a silicon wafer having oxide or nitride with 1 to 100 ohm-cm resistivity.

In step S2, forming a wavy pattern on the substrate, a wavy pattern is formed on the substrate 20, as shown in FIG. 8. A plurality of openings 210 are formed on the protective layer 21 of the substrate 20 using lithography and dry etching.

A plurality of grooves 200 are formed on the substrate 20 through the plurality of openings 210 using anisotropic etching, as shown in FIG. 9. The plurality of grooves 200 are inverted triangular grooves. An anisotropic etching solution of the anisotropic etching is 10 to 45 wt% Potassium hydroxide KOH at a temperature between 40 and 90 °C. Another anisotropic etching solution can be 5 to 25 wt% tetramethylammonium hydroxide TMAH or TMAOH at a temperature between 40 and 90°C.

As shown in FIG. 10, the protective layer 21 is removed. Each of the grooves 200 has acute angles 201 at the edge and bottom of the each groove 200.

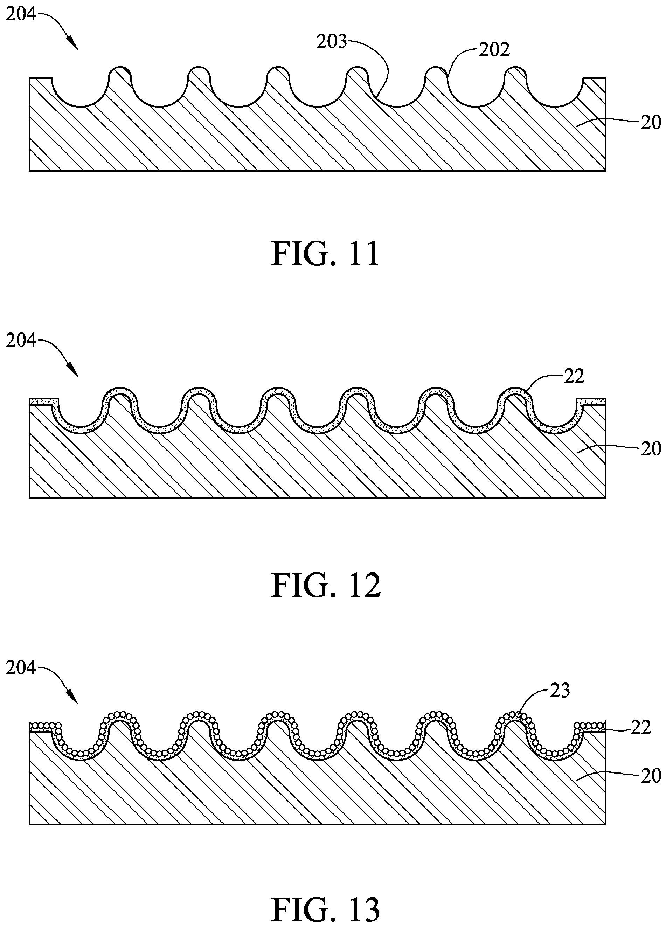

The protective layer 21 is removed after the grooves 200 are formed, as shown in FIG. 10. The substrate 20 is then immersed in the etching solution, and after being immersed for a few seconds, the acute angles 201 are formed into rounded corners 202, as shown in FIG. 11. Alternatively, the substrate 20 is treated with thermal oxidation to form the acute angles 201 into rounded corners 202, and to form the grooves 200 with inverted triangular shaped into grooves 203 with semicircular shaped. The grooves 203 with the rounded corners 202 form the wavy pattern 204.

In step S3, disposing a conductive substance on the wavy pattern, a conductive substance is disposed on the wavy pattern 204 using evaporating or sputtering of physical vapor deposition (PVD). A conductive layer 22 is formed on the wavy pattern 204 of the substrate 20 using evaporating or sputtering of physical vapor deposition, as shown in FIG. 12. The conductive layer 12 can be a chrome-copper (Cr-Cu) layer.

As shown in FIG. 13, a colloidal metal solution with conductive particles 23 and colloidal solution is disposed on the conductive layer 22 of the substrate 20, wherein the conductive particles 23 are formed on the conductive layer 12 after the colloidal solution dried.

FIG. 6 is a schematic view of the substrate having a conductive layer. The working electrode of electrochemical sensor includes a substrate 10, a wavy pattern 103, and a conductive substance, as shown in FIG. 6.

The wavy pattern 103 is formed on the substrate 10. The substrate 10 has the plurality of grooves 100, and each of the grooves 200 has rounded corners 102 at the edge of the each groove 200. The grooves 100 with the rounded corners 102 form the wavy pattern 103.

The conductive substance is disposed on the wavy pattern 103 of the substrate 10. The conductive substance is a conductive layer 12. The conductive layer 12 is formed on the wavy pattern 103 of the substrate 10.

Embodiment 3: A working electrode of electrochemical sensor of the Embodiment 3 is based on the Embodiment 1 of the present invention described above, so the same components use the same symbols. The working electrode of electrochemical sensor includes a substrate 10, a wavy pattern 103, and a conductive substance, as shown in FIG. 7.

In the Embodiment 3, the conductive substance includes a conductive layer 12 and a plurality of conductive particles 13. The conductive layer is disposed on a side of the substrate with the grooves. The conductive layer 12 and the conductive particles 13 are sequentially formed on the substrate 10.

In summary, the working electrode of the present invention has high precision and uses metal. The working electrode having nanometer particles of metal is fabricated into a chip. The surface of the detection region of the chip has a wavy pattern. The wavy pattern can increase the detection region to improve the accuracy of the detection.

## Claims

1. A method for manufacturing a working electrode of electrochemical sensor, comprising the steps of:
step S1, providing a substrate (10);
step S2, forming a wavy pattern (103) on the substrate (10); and
step S3, disposing a conductive substance on the wavy pattern (103).

2. The method for manufacturing a working electrode of electrochemical sensor as recited in claim 1, further comprising a step of forming a protective layer (11) on one side of the substrate (10).

3. The method for manufacturing a working electrode of electrochemical sensor as recited in claim 2, further comprising a step of forming a plurality of openings (110) on the protective layer (11) using lithography and dry etching.

4. The method for manufacturing a working electrode of electrochemical sensor as recited in claim 3, further comprising a step of forming a plurality of grooves (100) with semicircular shaped having acute angles (101) on the substrate through the plurality of openings (110) using isotropic etching, or forming a plurality of grooves (200) with inverted triangular having acute angles (201) on the substrate (10) through the plurality of openings (110) using anisotropic etching.

5. The method for manufacturing a working electrode of electrochemical sensor as recited in claim 4, further comprising the steps of:
removing the protective layer (11); and
forming the acute angles (101) into rounded corners (102) by immersing the substrate (10) in an isotropic etching solution for a few seconds, or by treating the substrate (10) with thermal oxidation to make the grooves (100) having the rounded corners (102), wherein the grooves (100) with the rounded corners (102) form the wavy pattern (103).

6. The method for manufacturing a working electrode of electrochemical sensor as recited in claim 5, wherein the step S3 of disposing the conductive substance on the wavy pattern (103) is to form a conductive layer (12) on the wavy pattern (103) using evaporating or sputtering of physical vapor deposition.

7. The method for manufacturing a working electrode of electrochemical sensor as recited in claim 6, further comprising a step of forming a colloidal metal solution with conductive particles (13) and colloidal solution on the conductive layer (12), wherein the conductive particles (13) are formed on the conductive layer (12) after the colloidal solution dried.

8. A working electrode of electrochemical sensor, comprising:
a substrate (10);
a wavy pattern (103), formed on the substrate (10); and
a conductive substance, disposed on the wavy pattern (103).

9. The working electrode of electrochemical sensor as recited in claim 8, wherein the wavy pattern (103) includes a plurality of grooves (100) formed on the substrate (10), and each of the grooves (100) has rounded corners (102) at the edge and bottom of the each groove (100).

10. The working electrode of electrochemical sensor as recited in claim 8, wherein the conductive substance includes a conductive layer (12), or the conductive substance includes a conductive layer (12) and a plurality of conductive particles (13) sequentially formed on the substrate (10), wherein the conductive layer is disposed on a side of the substrate with the grooves.
